(19) Europäisches Patentamt
European Patent Office
Office européen des brevets

(11) Veröffentlichungsnummer: **0 035 724**
**B1**

(12) **EUROPÄISCHE PATENTSCHRIFT**

(45) Veröffentlichungstag der Patentschrift: **18.05.83**

(51) Int. Cl.³: **C 07 C 17/18**, C 07 C 69/743, C 07 C 67/307

(21) Anmeldenummer: **81101466.1**

(22) Anmeldetag: **28.02.81**

(54) **Verfahren zur Herstellung von 1,1-Dihalogen-alkenen.**

(30) Priorität: **12.03.80 DE 3009487**

(43) Veröffentlichungstag der Anmeldung: **16.09.81 Patentblatt 81/37**

(45) Bekanntmachung des Hinweises auf die Patenterteilung: **18.05.83 Patentblatt 83/20**

(84) Benannte Vertragsstaaten: **AT BE CH DE FR GB IT LI NL**

(56) Entgegenhaltungen: **EP-A-0 002 849**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Priesnitz, Uwe, Dr., Severinstrasse 58, D-5650 Solingen (DE)**
Erfinder: **Riebel, Hans-Joachem, Dr., In der Beek 92, D-5600 Wuppertal (DE)**

**EP 0 035 724 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 1,1-Dihalogen-alkenen

Die Erfindung betrifft ein neues Verfahren zur Herstellung von bekannten 1,1-Dihalogen-alkenen.

Es ist bekannt, dass man bestimmte 1,1-Dihalogen-alkene, wie z.B. β,β-Difluor- oder β-Chlor-β-fluor-styrol, erhält, wenn man Carbonylverbindungen, wie z.B. Benzaldehyd, mit Alkalitrihalogenacetaten, wie z. B. Natrium-chlordifluoracetat bzw. Natrium-fluordichloracetat, in Gegenwart von Triphenylphosphin umsetzt (vgl. Tetrahedron Lett. 1964, 1461; ibid. 1968, 71; J. Org. Chem. 30 (1965), 1027).

Das bei diesem Verfahren zu verwendende Triphenylphosphin ist jedoch ein relativ teures Produkt. Die Wiedergewinnung des Triphenylphosphins aus dem bei der Umsetzung entstehenden Triphenylphosphinoxid ist mit unvertretbar hohem Aufwand verbunden. Es war daher erforderlich Wege zu suchen, wie dieses Verfahren kostengünstiger gestaltet werden kann.

Es war bekannt, dass Dihalogenalalkene durch Umsetzung von Trialkylaminophosphinen mit Tetrahalogenmethan und anschliessender Reaktion mit Carbonylverbindungen entstehen (EP-P 2849). Dabei ist es jedoch erforderlich, 2 Mol Trialkylaminophosphin auf 1 Mol Carbonylverbindung einzusetzen. Das Verfahren wird dadurch unwirtschaftlich.

Es wurde nun ein Verfahren zur Herstellung von 1,1-Dihalogen-alkenen der Formel I

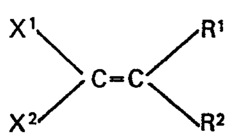

(I)

gefunden, in welcher

$R^1$ für Wasserstoff oder für einen gegebenenfalls substituierten Alkyl-, Alkenyl-, Alkinyl-, Cycloalkyl-, Aralkyl-, Aralkenyl- oder Aryl-Rest steht.

$R^2$ für einen gegebenenfalls substituierten Alkyl-, Alkenyl-, Alkinyl-, Cycloalkyl-, Aralkyl-, Aralkenyl- oder Aryl-Rest steht,

oder in welcher die beiden Reste $R^1$ und $R^2$ zusammen für eine gegebenenfalls verzweigte und/oder gegebenenfalls benzanellierte Kohlenwasserstoffkette stehen, und

$X^1$ und $X^2$ gleich oder verschieden sind und für Halogen stehen, dadurch gekennzeichnet, dass man Carbonylverbindungen der Formel II

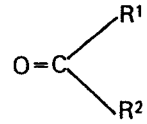

(II)

in welcher

$R^1$ und $R^2$ die oben angegebenen Bedeutungen haben,

mit Trihalogenacetaten der Formel III

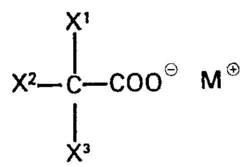

(III)

in welcher

$X^1$, $X^2$ und $X^3$ gleich oder verschieden sind und für Halogen stehen und

$M^{\oplus}$ für ein Alkalimetallion oder ein Erdalkalimetallionenäquivalent steht,

in Gegenwart von angenähert der äquimolaren Menge eines Phosphorsäuretrialkylesters (Trialkylphosphits) oder eines Phosphorigsäuretriamids und gegebenenfalls in Gegenwart eines Verdünnungsmittels bei Temperaturen zwischen 0 und 200°C umsetzt.

Es ist als überraschend anzusehen, dass man nach dem erfindungsgemässen Verfahren 1,1-Dihalogenalkene der Formel (I) in guten Ausbeuten erhält, da damit zu rechnen war, dass die Umsetzung von Carbonylverbindungen mit Trihalogenacetaten in Gegenwart von Trialkylphosphiten oder von Phosphorigsäuretriamiden weniger selektiv als bei Verwendung von Triphenylphosphin ablaufen würde.

Als Vorteile des erfindungsgemässen Verfahrens sind zu erwähnen, dass Trialkylphosphite und Phosphorigsäuretriamide preiswerter sind als Triphenylphosphin. Das neue Verfahren ist somit kostengünstiger als die nächstliegende aus dem Stand der Technik bekannte Synthesemethode.

Verwendet man als Ausgangsstoffe beispielsweise 3-Formyl-2,2-dimethyl-cyclopropan-1-carbonsäuremethylester, Natrium-trichloracetat und Trimethylphosphit, so kann die Reaktion dieser Verbindungen durch folgendes Formelschema skizziert werden:

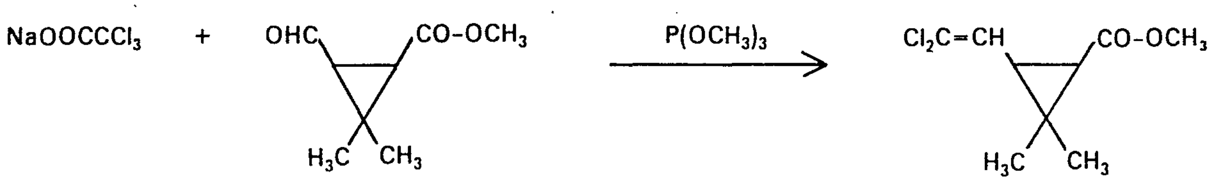


Die als Ausgangsstoffe zu verwendenden Carbonylverbindungen sind durch Formel (II) definiert.

Vorzugsweise stehen darin

$R^1$ für Wasserstoff für gegebenenfalls halogensubstituiertes $C_1$–$C_5$-Aklyl, für gegebenenfalls halogensubstituiertes Benzyl oder Phenylethyl, oder für gegebenenfalls durch Halogen, $C_1$–$C_4$-Alkyl, $C_1$–$C_4$-Alkoxy, Methylendioxy, Cyano und/

oder Nitro substituiertes Phenyl, und für gegebenenfalls halogen-substituiertes $C_1$–$C_5$-Alkyl, für $C_2$–$C_5$-Alkinyl, für gegebenenfalls halogen-substituiertes Benzyl oder Phenylethyl, für gegebenenfalls durch Halogen, $C_1$–$C_4$-Alkyl, $C_1$–$C_4$-Alkoxy, Methylendioxy, Cyano und/oder Nitro substituiertes Phenyl, für gegebenenfalls halogen-substituiertes Styryl oder für den Rest

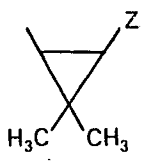

worin Z für Acetyl, Cyano, Carbamoyl, $C_1$–$C_4$-Alkoxycarbonyl oder für COOM steht, wobei M für Wasserstoff, ein Alkalimetall, ein Erdalkalimetalläquivalent oder einen Ammoniumrest steht.

Als Ausgangsstoffe besonders bevorzugt sind diejenigen Verbindungen der Formel (II), in welcher

$R^1$ für Wasserstoff steht und

$R^2$ für $C_2$–$C_5$-Alkenyl oder den Rest

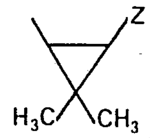

steht, worin

Z für Cyano, Acetyl, Carboxy, $C_1$–$C_4$-Alkoxycarbonyl oder für COOM steht, wobei M für Natrium oder Kalium steht.

Als Beispiele für die Ausgangsverbindungen der Formel (II) seien genannt:

β,β-Dimethyl-acrolein, 3-Formyl-2,2-dimethyl-1-cyanocyclopropan, 3-Formyl-2,2-dimethyl-1-acetyl-cyclopropan, 3-Formyl-2,2-dimethyl-cyclopropan-1-carbonsäure und der Natriumsalz sowie 3-Formyl-2,2-dimethyl-cyclopropan-1-carbonsäure-methylester, -ethylester, -n-propylester, -iso-propylester, -n-butylester, -iso-butylester, -sek.-butylester und -tert.-butylester.

Die Verbindungen der Formel (II) sind teilweise bekannt (vergleiche Synthesis 1975, 535–536; Tetrahedron Lett. 1976, 1979–1982). Die besonders bevorzugten Cyclopropanderivate der Formel (II) sind teilweise noch nicht in der Literatur beschrieben. Man erhält diese Verbindungen nach an sich bekannten Verfahren. Ein Syntheseweg ist in nachstehendem Formelschema skizziert: (R steht für $C_1$–$C_4$-Alkyl)

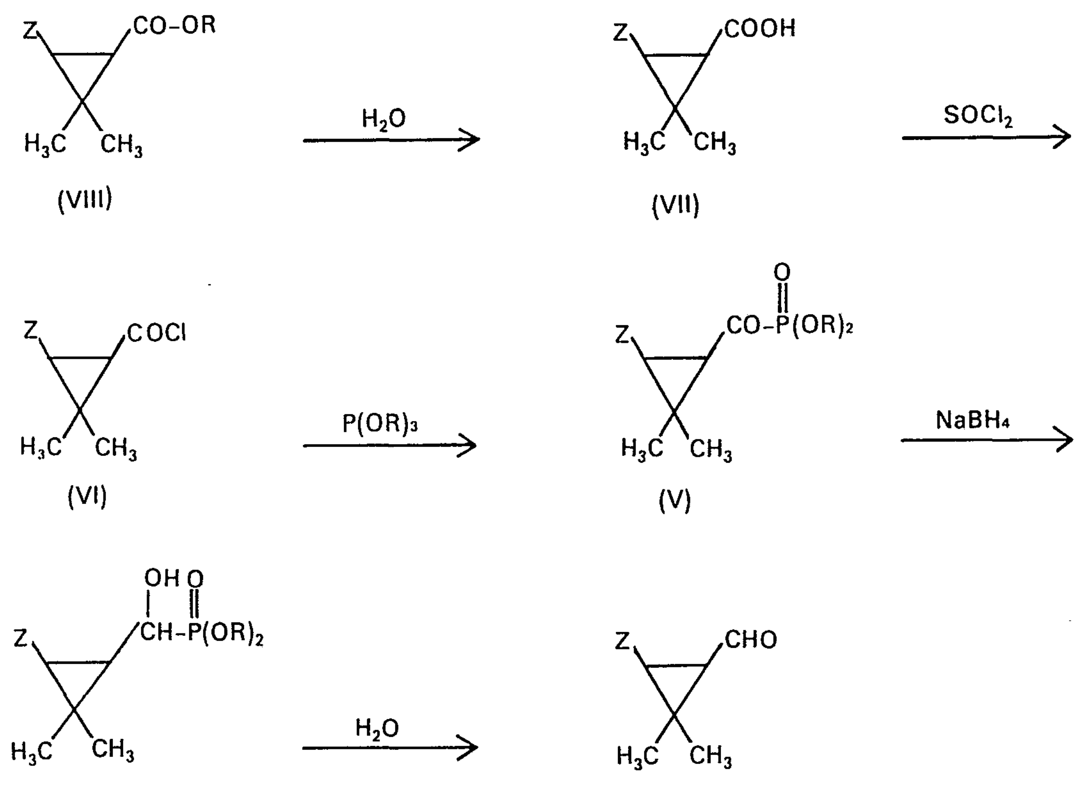


(VIII) (VII) (VI) (V) (IV) (IIa)

Durch Hydrolyse von bekannten Cyclopropancarbonsäureestern der Formel (VIII) (vgl. J. Org. Chem. 32, (1967), 3351–3355; Bull. Soc. Chim. Belg. 87 (1978), 721–732; Tetrahedron Lett. 1978 1847–1850), beispielsweise durch Umsetzung mit wässrig-alkoholischer Kalilauge bei Temperaturen zwischen 20 und 100°C und anschliessendes Ansäuern erhält man die Carbonsäuren der Formel (VII). Diese können durch Umsetzen mit Halogenierungsmitteln, wie z. B. Thionylchlorid, bei Temperaturen zwischen 20 und 80°C in die Säurechloride der Formel (VI) umgewandelt werden.

Durch Umsetzung der Säurechloride (VI) mit Trialkylphosphiten bei Temperaturen zwischen -20 und +150°C, vorzugsweise zwischen 0 und 120°C, erhält man die Cyclopropanoylphosphonsäureester der Formel (V) (vgl. J. Am. Chem. Soc. 86 (1964), 3862–3866; Methoden der organischen Chemie (Houben-Weyl-Müller), 4. Auflage, Band 12/1, S. 453, Georg-Thieme-Verlag, Stuttgart 1963). Zur Isolierung und Reinigung der Produkte wird, gegebenenfalls unter vermindertem Druck destilliert.

Die α-Hydroxy-phosphonsäureester der Formel (IV) erhält man durch Reduktion der Oxoverbindungen der Formel (V) mit Natriumtetrahydridoborat, gegebenenfalls unter Verwendung eines Verdünnungsmittels, wie z. B. Wasser oder wässriges Methanol, bei Temperaturen zwischen -20 und +50°C und Halten des pH-Wertes zwischen 5 und 8 durch Zugabe eines Puffermittels, wie z. B. Natriumhydrogenphosphat (vgl. Chem. Ber. 103 (1970), 2984–2986). Zur Aufarbeitung extrahiert man mit einem mit Wasser nicht mischbaren Lösungsmittel, wie z. B. Methylenchlorid, trocknet die Extrakte, filtriert und destilliert das Lösungsmittel unter vermindertem Druck ab.

Aus den α-Hydroxy-phosphonsäureestern der Formel (IV) können die entsprechenden Aldehyde der Formel (IIa) durch Behandeln mit Natronlauge bei Temperaturen zwischen 0 und 100°C, vorzugsweise zwischen 10 und 50°C hergestellt werden (vgl. Chem. Ber. 103 (1970), 2984–2986).

Alternativ zum oben skizzierten Herstellungsverfahren erhält man Aldehyde der Formel (IIa) auch durch Umsetzung von Säurechloriden der Formel (VI) mit Lithiumtritert.-butoxy-hydridoaluminat, welches man gegebenenfalls in situ aus Lithium-tetrahydridoaluminat und tert.-Butanol hergestellt hat, gegebenenfalls in Gegenwart eines Verdünnungsmittels, wie z. B. Tetrahydrofuran, bei Temperaturen zwischen −100 und +100°C, vorzugsweise zwischen -80 und +50°C. Zur Aufarbeitung wird in eine Mischung aus Salzsäure und Eiswasser gegossen und mit einem mit Wasser nicht mischbaren Lösungsmittel, wie z. B. Diethylether, extrahiert. Die Extrakte werden getrocknet, filtriert und eingeengt. Zur Reinigung des Rohproduktes wird gegebenenfalls destilliert.

Die weiter als Ausgangsstoffe einzusetzenden Trihalogenacetate sind durch Formel (III) definiert. Vorzugsweise stehen darin

$X^1$, $X^2$ und $X^3$ unabhängig von einander für Fluor, Chlor oder Brom, insbesondere für Chlor, und

$M^\oplus$ steht für ein Natrium- oder Kalium-ion.

Als Beispiel seien Natrium-trichloracetat, Natrium-chlordifluoracetat und Natrium-fluor-dichloracetat genannt.

Die Verbindungen der Formel (III) sind bekannt.

Als Beispiel für die beim erfindungsgemässen Verfahren zu verwendenden Phosphorigsäuretrialkylester bzw. Phosphorigsäureamide seien Trimethylphosphit, Triethylphosphit, Tripropylphosphit und Phosphorigsäure-tris-dimethylamid genannt.

Das erfindungsgemässe Verfahren wird vorzugsweise unter Verwendung von Verdünnungsmitteln durchgeführt. Als solche kommen praktisch alle inerten organischen Lösungsmittel insbesondere aprotisch polare Solventien in Frage. Hierzu gehören Ether, wie z. B. Glycoldimethylether, Diglycoldimethylether, Tetrahydrofuran und Dioxan, Carbonsäureamide, wie z. B. Dimethylformamid, Dimethylacetamid und N-Methylpyrrolidon, Sulfoxide, wie z. B. Dimethylsulfoxid, Phosphorsäureamide, wie z. B. Hexamethylphosphorsäuretriamid, sowie Nitrile, wie z. B. Acetonitril und Propionitril.

Die Reaktionstemperatur wird zwischen 0 und 200°C, vorzugsweise zwischen 10 und 180°C, bei Verwendung von Trialkylphosphiten insbesondere zwischen 140 und 160°C, bei Verwendung von Phosphorigsäureamiden insbesondere zwischen 10 und 50°C gehalten. Das Verfahren wird im allgemeinen bei Normaldruck oder einem dem Dampfdruck des jeweiligen Verdünnungsmittels bei der Reaktionstemperatur entsprechender Druck durchgeführt.

Je Mol Carbonylverbindung der Formel (III) werden 0,9 bis 1,2 Mol, vorzugsweise 0,95 bis 1,1 Mol Trihalogenacetat der Formel (II) und 0,9 bis 1,2 Mol, vorzugsweise 0,95 bis 1,1 Mol Phosphorigsäureester bzw. -amid eingesetzt.

Zur Durchführung des erfindungsgemässen Verfahrens werden bei Verwendung von Phosphorigsäuretrialkylestern vorzugsweise alle Reaktionskomponenten bei Raumtemperatur (ca. 20°C) in einem Verdünnungsmittel vermischt und das Reaktionsgemisch wird unter Rühren mehrere Stunden lang auf 140 bis 160°C erhitzt.

Bei Verwendung von Phosphorigsäuretriamiden werden vorzugsweise die Ausgangsstoffe der Formel (II) und (III) in einem Verdünnungsmittel vorgelegt und bei 10 bis 50°C das Phosphorigsäureamid langsam zudosiert, wonach das Reaktionsgemisch noch einige Stunden bei Raumtemperatur gerührt wird.

Die Aufarbeitung kann nach üblichen Methoden durchgeführt werden, beispielsweise, indem man das Reaktionsgemisch mit Wasser verdünnt, mit einem mit Wasser praktisch nicht mischbaren organischen Lösungsmittel, wie z. B. Methylenchlorid, extrahiert, die organische Phase trocknet, filtriert und eindampft. Die im Rückstand verbleibenden Produkte können auf übliche Weise, z. B. durch Vakuumdestillation, gereinigt werden.

Die nach dem erfindungsgemässen Verfahren herzustellenden 1,1-Dihalogen-alkene können zum Teil als Zwischenprodukte zur Herstellung von insektizid und akarizid wirksamen Pyrethroiden verwendet werden (vgl. DE - OS 2 326 077).

Beispiel 1:

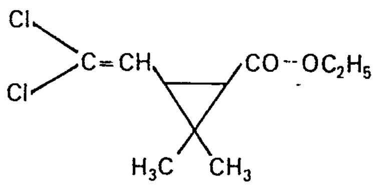

Eine Mischung von 17,0 g (0,1 Mol) 3-Formyl-2,2-dimethyl-cyclopropan-1-carbonsäureethylester 16,6g (0,1 Mol) Triethylphosphit und 18,5 g (0,1 Mol) Trichloressigsäurenatriumsalz in 100 ml Diglyme wird unter Argon 6 Stunden auf 150°C erhitzt. Dann wird das Reaktionsgemisch in Wasser gegeben. Es folgt eine Extraktion mit 2 mal je 100 ml Methylenchlorid. Die vereinigten Methylenchloridextrakte werden über Natriumsulfat getrocknet. Man erhält 3-(2,2-Dichlorvinyl)-2,2-dimethyl-cyclopropan-1-carbonsäure-ethylester als Rohprodukt in einer Ausbeute von 73% der Theorie.

Beispiel 2:

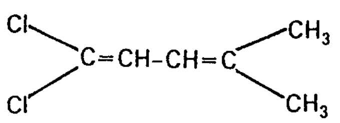

Zu einer Mischung aus 9,2 g (0,05 Mol) Trichloressigsäurenatriumsalz, 4,2 g (0,05 Mol) β,β-Dimethylacrolein und 100 ml Diethylenglykoldimethylether tropft man 8,2 g (0,05 Mol) Phosphorigsäure-tris-dimethylamid. Die Temperatur steigt dabei bis ca. 45°C an. Man rührt das Gemisch 3 Stunden nach, gibt 200 ml Wasser zu und extrahiert 2 mal mit je 100 ml Methylenchlorid. Die vereinigten organischen Phasen werden mit 50 ml 10%iger Natronlauge und 2 mal mit je 50 ml Wasser gewaschen, über Natriumsulfat getrocknet und im Vakuum eingedampft. Der Rückstand wird im Vakuum destilliert. Man erhält so 7,3 g (77% der Theorie) 1,1-Dichlor-4,4-dimethylbutadien in Form einer farblosen Flüssigkeit mit dem Siedepunkt 54–56°C/10 Torr.

**Patentanspruch**

1. Verfahren zur Herstellung von 1,1-Dihalogenalkenen der Formel I

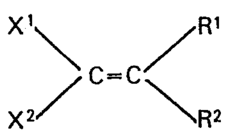
(I)

in welcher
$R^1$ für Wasserstoff oder für einen gegebenenfalls substituierten Alkyl-, Alkenyl-, Alkinyl-, Cycloalkyl-, Aralkyl-, Aralkenyl- oder Aryl-Rest steht,
$R^2$ für einen gegebenenfalls substituierten Alkyl-, Alkenyl-, Alkinyl-, Cycloalkyl-, Aralkyl-, Aralkenyl- oder Aryl-Rest steht,
oder in welcher die beiden Reste $R^1$ und $R^2$ zusammen für eine gegebenenfalls verzweigte und/oder gegebenenfalls benzannellierte Kohlenwasserstoffkette stehen, und
$X^1$ und $X^2$ gleich oder verschieden sind und für Halogen stehen,
dadurch gekennzeichnet, dass man Carbonylverbindungen der Formel II

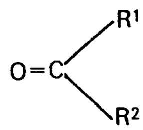
(II)

in welcher
$R^1$ und $R^2$ die oben angegebenen Bedeutungen haben,
mit Trihalogenacetaten der Formel III

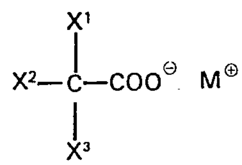
(III)

in welcher
$X^1$, $X^2$ und $X^3$ gleich oder verschieden sind und für Halogen stehen und
$M^{\oplus}$ für ein Alkalimetallion oder ein Erdalkalimetallionenäquivalent steht,
in Gegenwart von angenähert der äquimolaren Menge eines Phosphorigsäuretrialkylesters (Trialkylphosphits) oder eines Phosphorigsäuretriamids und gegebenenfalls in Gegenwart eines Verdünnungsmittels bei Temperaturen zwischen 0 und 200°C umsetzt.

**Claim**

1. Process for the preparation of 1,1-dihalogenoalkenes of the formula I

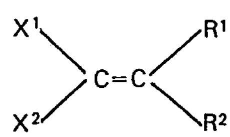
(I)

in which
$R^1$ represents hydrogen or an optionally substituted alkyl, alkenyl, alkynyl, cycloalkyl, aralkyl, aralkenyl or aryl radical and
$R^2$ represents an optionally substituted alkyl, alkenyl, alkynyl, cycloalkyl, aralkyl, aralkenyl or aryl radical,
or in which the two radicals $R^1$ and $R^2$ together represent an optionally branched and/or optionally benzo-fused hydrocarbon chain, and
$X^1$ and $X^2$ are identical or different and represent halogen,
characterised in that carbonyl compounds of the formula II

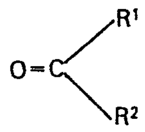

(II)

in which
$R^1$ and $R^2$ have the abovementioned meanings, are reacted with trihalogenoacetates of the formula III

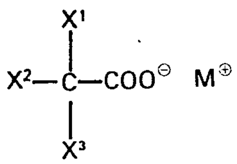

(III)

in which
$X^1$, $X^2$ and $X^3$ are identical or different and represent halogen and
$M^{\oplus}$ represents an alkali metal ion or one equivalent of an alkaline earth metal ion, in the presence of approximately the equimolar amount of a phosphorous acid trialkyl ester (trialkyl phosphite) or of a phosphorous acid triamide, and if appropriate in the presence of a diluent, at temperatures between 0 and 200°C.

**Revendication**

Procédé de préparation de 1,1-dihalogénoalcènes de formule I:

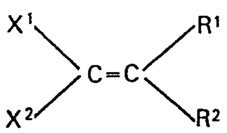

(I)

dans laquelle
$R^1$ représente un atome d'hydrogène ou un groupe alkyle, alcényle, alcinyle, cycloalkyle, aralkyle, aralcényle ou aryle éventuellement substitué,
$R^2$ représente un groupe alkyle, alcényle, alcinyle, cycloalkyle, aralkyle, aralcényle ou aryle éventuellement substitué,
ou dans laquelle les deux radicaux $R^1$ et $R^2$ ensemble représentent une chaîne hydrocarbonée éventuellement ramifiée et/ou benzo-condensée, et
$X^1$ et $X^2$ sont identiques ou différents et représentent chacun un atome d'halogène,
caractérisé en ce qu'on fait réagir des composés carbonyl de formule II:

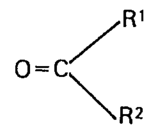

(II)

dans laquelle
$R^1$ et $R^2$ ont les significations indiquées ci-dessus, avec des trihalogénacétates de formule III:

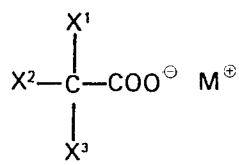

(III)

dans laquelle
$X^1$, $X^2$ et $X^3$ sont identiques ou différents et représentent chacun un atome d'halogène, et
$M^{\oplus}$ représente un ion d'un métal alcalin ou un équivalent d'un ion de métal alcalino-terreux,
en présence d'un ester trialkylique d'acide phosphoreux (phosphite de trialkyle) ou d'un triamide d'acide phosphoreux en une quantité à peu près équimolaire et éventuellement en présence d'un diluant, à des températures comprises entre 0 et 200°C.